# EUROPÄISCHE PATENTANMELDUNG

(11) **EP 1 077 265 A1**
(43) Veröffentlichungstag der Anmeldung: **21.02.2001**
(21) Anmeldenummer: 99116340.3
(22) Anmeldetag: 19.08.1999
(51) Int. Cl.: C12Q 1/68

(54) **Verfahren zur genotypischen Klassifizierung von Bakterien**

(71) Anmelder: Merlin Gesellschaft für mikrobiologische Diagnostika mbH, 53332 Bornheim (DE)
(72) Erfinder: Heisig, Peter, D-53859 Niederkassel (DE); Fuchs-Gomez, Yolanda, W.Lynnwood, WA98037 (US)
(74) Vertreter: Meyers, Hans-Wilhelm, Dr.Dipl.-Chem.

(57) **Zusammenfassung**

Das Verfahren zur genotypischen Klassifizierung von Bakterien, dadurch gekennzeichnet, daß
Sequenzen von Teilbereichen mindestens eines Gens ausgewählt aus der Gruppe gyrA, gyrB, parC und par E bestimmt werden und
die Klassifizierung durch Vergleich mit den bekannten Sequenzen der jeweiligen Gene von Bakterien erfolgt, wobei
   - im Falle des gyrA-Gens die Codons für die Aminosäuren Gly-81, Ser-83, Ala-84, Asp-87 gemäß der Numerierung des E.coli-gyrA-Gens für den Vergleich unberücksichtigt bleiben;
   - im Falle des parC-Gens die Codons für die Aminosäuren Gly-78, Ser-80, Ala-81, Glu-84 gemäß der Numerierung des E.coli-parC-Gens für den Vergleich unberücksichtigt bleiben;
   - im Falle des gyrB-Gens die Codons für die Aminosäuren Asp-426 und Lys-447 gemäß der Numerierung des E.coli-gyrB-Gens für den Vergleich unberücksichtigt bleiben und
   - im Falle des parE-Gens die Codons für die Aminosäuren Asp-420 und Lys-441 gemäß der Numerierung des E.coli-parE-Gens für den Vergleich unberücksichtigt bleiben.

## Beschreibung

Die vorliegende Erfindung betrifft ein Verfahren zur genotypischen Klassifizierung von Bakterien.

Das üblicherweise eingesetzte Verfahren zur Klassifizierung von Bakterien, insbesondere in der klinischen Diagnostik, basiert auf phänotypischen Unterschieden der Organismen. Die Klassifizierung erfolgt über Aktivitätsmuster verschiedener metabolischer Enzyme, wie dies zum Beispiel in Holt, J.G. (Hrsg.): Bergey's Manual of Systematic Bacteriology, Vol. 1-4, Williams & Wilkins, Baltimore, 1984, 1986, 1989 beschrieben ist.

Die sich hieraus ergebene Klassifizierung der Bakterien ist jedoch willkürlich und künstlich. Die Standardisierung ist durch die Notwendigkeit einer Quantifizierung und hohe Anforderung an die Spezifität der enzymatischen Reaktionen erschwert.

Die ersten Ansätze zu einer genotypischen Klassifizierung beruhten auf 16S-rRNA Sequenzhomologien, die hierbei als repräsentativ für das Gesamtgenom angesehen wurden. Andere universell verbreitete Gene, die zur Klassifizierung eingesetzt wurden, sind die Gene für den Elongation Factor G (EF-G) und protontransportierende ATPasen (Olsen & Woese, FASEB J. 7 (1993) 113-223). Die genotypische Klassifizierung auf Basis von 16S-rRNA Sequenzhomologien stellt dabei eine natürliche, phylogenetische, systematische Bakteriologie zur Verfügung (Woese et al., Proc. Natl. Acad. Sci. USA 87 (1990) 4567-4579). Zur Überwindung der Schwierigkeiten, die durch die Vielfalt der Proben und die Schwierigkeiten der Kultivierung unbekannter Organismen entstehen, wurden von verschiedenen Forschungsgruppen eine Vielzahl von Hybridisierungstechniken (in situ Hybridisierung, Gesamtzell-Hybridisierung, quantitativer Dot Blot, Southern Blot unter Verwendung von isolierter, komplementärer rDNA) eingesetzt. In Abhängigkeit von der Spezifität der jeweiligen Oligonukleotid-Sonde konnten Klassifizierungen auf dem Niveau der Domäne, der Abteilung, des Genus oder sogar auf Speziesniveau erreicht werden (Amann et al, Microbiol. Rev. 59 (1995) 143-169). Der Ansatz wurde erfolgreich zur Klassifizierung von neuen pathogenen Bakterien eingesetzt, die nicht kultiviert werden konnten und mit spezifischen Erkrankungen in Verbindung gebracht wurden (Fredericks & Relman, Clin. Microbiol. Rev. 9 (1996) 18-33). Trotzdem ist dieses Verfahren nicht zur routinemäßigen Charakterisierung von beispielsweise Enterobakterien geeignet, da diese auf Speziesniveau keine speziesspezifischen Variationen in der 16S-rRNA enthalten.

Ein Gen zur schnellen und fehlerfreien Identifikation von Bakterien auf Speziesniveau sollte die folgenden Kriterien erfüllen:
1. Das Gen ist universell in allen Bakterien vorhanden;
2. Die Variationen der Sequenz des Gens sind stabil;
3. Es liegen in einem Stamm nur identische Kopien des Gens vor;
4. Variationen in der Sequenz des Gens sind klein zwischen einzelnen Stämmen einer Spezies im Vergleich zu den Variationen zwischen verschiedenen Spezies;
5. Das Gen enthält speziesspezifische Variationen;
6. Das Gen ist konserviert, so daß die Detektion in verschiedenen Spezies möglich ist.

Aus den erhältlichen Daten läßt sich annehmen, daß die Klassifizierung über 16S-rRNA die Kriterien 1, 2, 4 und 6 erfüllt, wobei jedoch in einigen Spezies unterschiedliche Kopien der ribosomalen Gene beobachtet wurden (Kriterium 3) *(E.coli:* Cilia et al., Mol. Biol. Evol. 13 (1995) 451-461; *Mycobacterium celatum:* Reischl et al., J. Clin. Microbiol. 36 (1998) 1761-1764; *Mycobacterium fortuitum:* Kirschner et al., J. Clin. Microbiol. 30 (1992) 2772-2775; *Mycobacterium ulcerans:* Portaels et al., J. Clin. Microbiol. 34 (1996) 962-965, *Mycobacterium terrae:* Ninet et al., J. Clin. Microbiol. 34 (1996) 2531-2536).

Darüber hinaus ist die Sensitivität der 16S-rRNA Methode für die Familie der Entero-bakteriaceae gering, da es keine speziesspezifischen Variationen der Gene gibt (Kriterium 5) (Fox et al., Int. J. Syst. Bacteriol. 42 (1992) 166-170; Stackebrandt and Goebel, Int. J. Syst. Bacteriol. 44 (1994) 846-849).

Als ein alternativer Ansatz zur Klassifizierung auf der Basis der 16S-rRNA-Gene wurden kürzlich Teilsequenzen des rpoB-Gens vorgeschlagen, mit denen Enterobak-terienspezies unterschieden werden können (Mollet et al., Mol. Microbiol. 26 (1997) 1005-1011). Abgesehen von der nur begrenzten Verfügbarkeit von Sequenz-Informationen über das rpoB-Gen ist dieses trotzdem erfolgreich zur Klassifizierung von Archaea und Bacteria eingesetzt worden (Pühler et al., Proc. Natl. Acad. Sci. USA 86 (1989) 4569-4573; Klenk & Zillig, J. Mol. Evol. 38 (1994) 420-432; Rowland et al., Biochem. Soc. Trans. 21 (1992) 40S).

Daher ist anzunehmen, daß das rpoB-Gen auch die Kriterien 1, 2, 4 und 6 der oben genannten Aufstellung erfüllt. Bisher gibt es keine Berichte, daß mehr als eine Kopie des rpoB-Gens in einem Stamm vorliegt, daher wird auch das Kriterium 3 erfüllt.

Im Gegensatz zu den 16S-rRNA-Genen, die kein Protein kodieren, ist das Alignment von einem unbekannten rpoB-Gen durch die Existenz eines Leserahmens für das konservierte Protein erleichtert. Bei der Untersuchung von klinisch relevanten Bakterien, die häufig Mutationen im Zusammenhang mit der Rifampin-Resistenz tragen, treten jedoch nicht nur Punktmutationen, sondern auch Deletionen und Insertionen (Musser, Clin. Microbiol. Rev. 8 (1995) 496-514) auf, die die Anwendung konventioneller Hybridisierungstechniken zur Identifizierung erschweren. Die automatische Bestimmung und der Vergleich von DNA-Sequenzen aus einer Vielzahl von Isolaten ist nicht einfach und erschwert die Anwendung des Verfahrens in der Routinediagnostik.

Die Aufgabe der vorliegenden Erfindung ist es, ein Verfahren zur genotypischen Klassifizierung bereitzustellen, daß die oben genannten Nachteile der bekannten Verfahren überwindet.

Gelöst wird die Aufgabe durch ein Verfahren mit den Merkmalen des Patentanspruchs 1.

Das erfindungsgemäße Verfahren zur genotypischen Klassifizierung von Bakterien ist dadurch gekennzeichnet, daß
Sequenzen von Teilbereichen mindestens eines Gens ausgewählt aus der Gruppe gyrA, gyrB, parC und parE bestimmt werden und
die Klassifizierung durch Vergleich mit den bekannten Sequenzen der jeweiligen Gene von Bakterien erfolgt, wobei
   - im Falle des gyrA-Gens die Codons für die Aminosäuren Gly-81, Ser-83, Ala-84, Asp-87 gemäß der Numerierung des E.coli-gyrA-Gens für den Vergleich unberücksichtigt bleiben;
   - im Falle des parC-Gens die Codons für die Aminosäuren Gly-78, Ser-80, Ala-81, Glu-84 gemäß der Numerierung des E.coli-parC-Gens für den Vergleich unberücksichtigt bleiben;
   - im Falle des gyrB-Gens die Codons für die Aminosäuren Asp-426 und Lys-447 gemäß der Numerierung des E.coli-gyrB-Gens für den Vergleich unberücksichtigt bleiben und
   - im Falle des parE-Gens die Codons für die Aminosäuren Asp-420 und Lys-441 gemäß der Numerierung des E.coli-parE-Gens für den Vergleich unberücksichtigt bleiben.

Das neue Verfahren beruht auf der Analyse von spezies- und subspeziesspezifischen DNA-Sequenzvariationen in konservierten Bereichen der Gene gyrA, gyrB, parC und parE, die für Untereinheiten der bakteriellen Topoisomerase II (Gyrase) und Topoisomerase IV codieren. Die beobachteten Variationen betreffen im wesentlichen die dritte "wobble" Position verschiedener Codons in diesen Regionen, die daher in den meisten Fällen nicht die Aminosequenz des exprimierten Proteins ändern.

Innerhalb der Gene befinden sich auch die Abschnitte (quinolone resistance-determining regions, QRDR), in denen alle bislang mit Chinolonresistenz assoziierten Mutationen enthalten sind. Die entsprechenden Resistenz-Mutationen sind häufig Punkt-Mutationen, die nur einzelne Aminosäuren betreffen. Im Falle des gyrA-Gens sind dies insbesondere Glycin-81, Serin-83, Alanin-84 und Aspartat-87 bezogen auf die Numerierung des E.coli-gyrA-Gens. Für das parC-Gen sind im wesentlichen die Aminosäuren Glycin-78, Serin-80, Alanin-81 und Glutamat-84 an der ChinolonResistenz beteiligt. Die entsprechenden Aminosäuren sind im Falle des gyrB-Gens Aspartat-426 und Lysin-447 bzw. im Falle des parE-Gens Aspartat-420 und Lysin-441. Da diese Mutationen nicht speziesspezifisch sind, bleiben sie erfindungsgemäß bei der Klassifizierung der Bakterien unberücksichtigt. Soweit bekannt ist, sind die Gene gyrA und gyrB in allen bislang untersuchten Bakterien (parC, ParE - noch - nicht in Mycobakterien) vorhanden, liegen nur als einfache Kopie vor und sind auf Proteinebene in den angegebenen Bereichen hochkonserviert. Es wird bevorzugt, daß zumindest zwei Gene gleichzeitig zur Klassifizierung eingesetzt werden.

Die Bestimmung der Sequenzen erfolgt vorzugsweise zunächst über einen Amplifizierungsschritt für Teilbereiche der Gene. Dazu werden beispielsweise mit Hilfe der Polymerase-Chain-Reaction unter Verwendung von Primerpaaren, die an hochkonservierte, flankierende Bereiche der Teilbereiche des Gens binden, die für die Klassifizierung relevanten Bereiche amplifiziert. Die Bestimmung kann entweder durch Sequenzierung der amplifizierten Bereiche oder durch Hybridisierung der amplifizierten Bereiche mit speziesspezifischen Oligonukleotiden, gefolgt von beispielsweise spektroskopischer Analyse der Hybridisierung, erfolgen. Die speziesspezifischen Oligonukleotid-Sonden weisen dabei bevorzugt eine Länge von 8 bis 14 Nukleotiden auf. Vorzugsweise kann für das erfindungsgemäße Verfahren die Array-Technologie eingesetzt werden, bei der die komplementären Oligonukleotide an einer Oberfläche fixiert sind, wobei die Analyse durch Markierung der Oligonukleotide mit verschiedenen Fluoreszenzfarbstoffen erleichtert werden kann.

Das erfindungsgemäße Verfahren erlaubt nicht nur die genotypische Klassifizierung von Bakterien, sondern darüber hinaus eine weitere Unterteilung auf Subspeziesniveau, wobei auch epidemiologische Beziehungen zwischen verschiedenen Isolaten aufgezeigt werden können. Darüber hinaus kann gleichzeitig das Vorliegen von Chinolon-Resistenz-Mutationen der isolierten Bakterien nachgewiesen werden.

Beansprucht werden daher auch Nukleinsäuren mit der Seq ID-Nr. 1 bis 13 sowie Fragmente der Nukleinsäuren mit einer Länge von mindestens acht Nukleotiden. Diese Nukleinsäuren und ihre Fragmente können als Sonden zur Klassifizierung von Bakterien verwendet werden.

Gegenstand der Erfindung ist weiterhin eine Zusammensetzung, die mindestens zwei Nukleinsäuren, die spezifisch für Sequenzen von Teilbereichen von Genen, ausgewählt aus der Gruppe gyrA, gyrB, parC und parE von Bakterien-Spezies sind sowie eine Analysevorrichtung zur Klassifizierung von Bakterien, wobei die Analysevorrichtung Nukleinsäuren enthält, die spezifisch für Sequenzen von Teilbereichen von Genen, ausgewählt aus der Gruppe gyrA, gyrB, parC und parE von Bakterien-Spezies sind. Auch die Verwendung dieser Analysevorrichtung oder der oben genannten Zusammensetzung zur analytischen oder diagnostischen Klassifizierung von Bakterien ist Gegenstand der Erfindung.

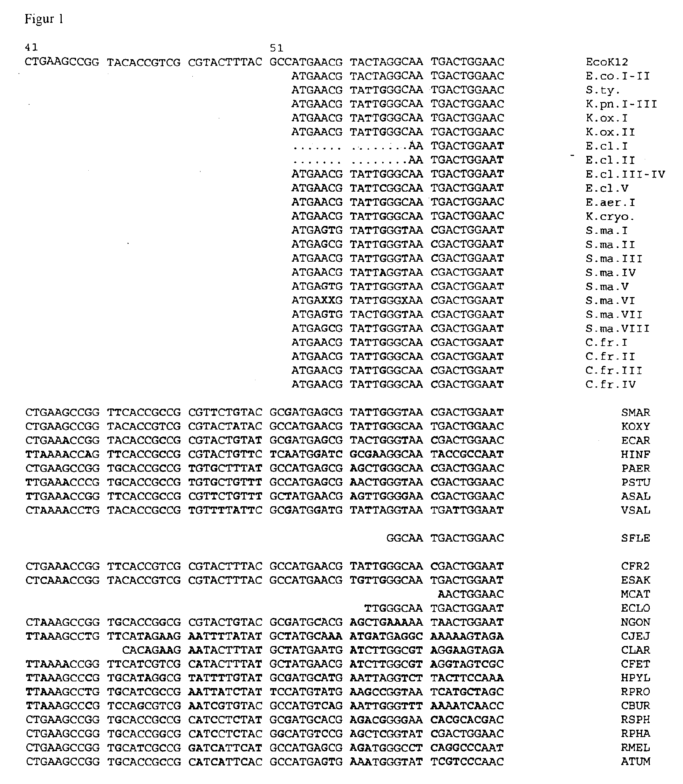

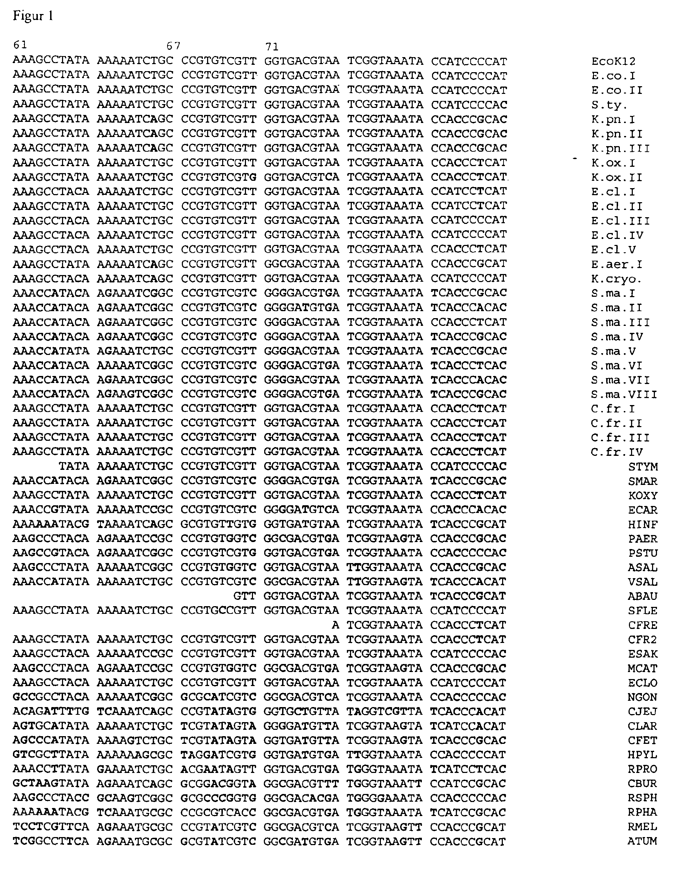

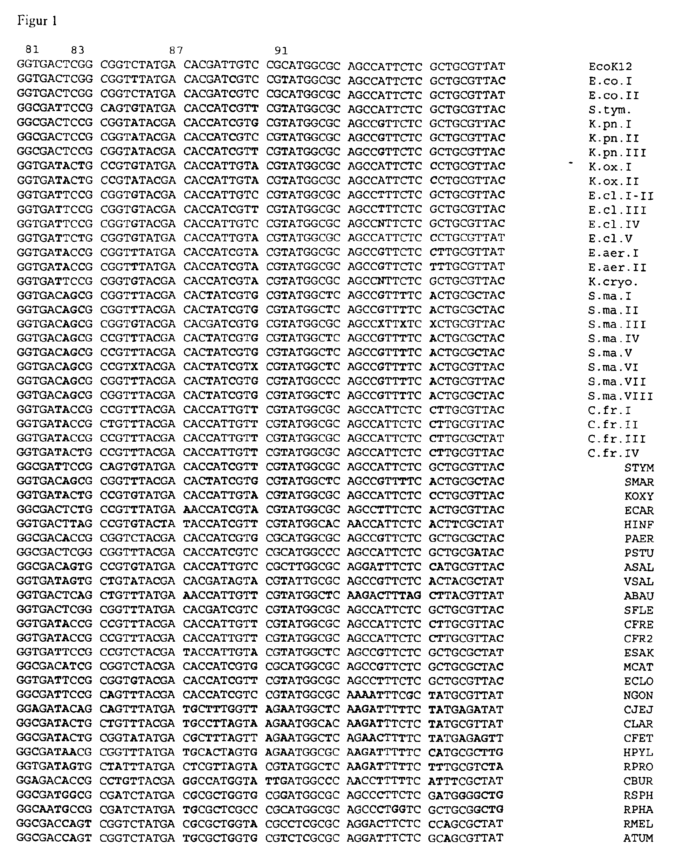

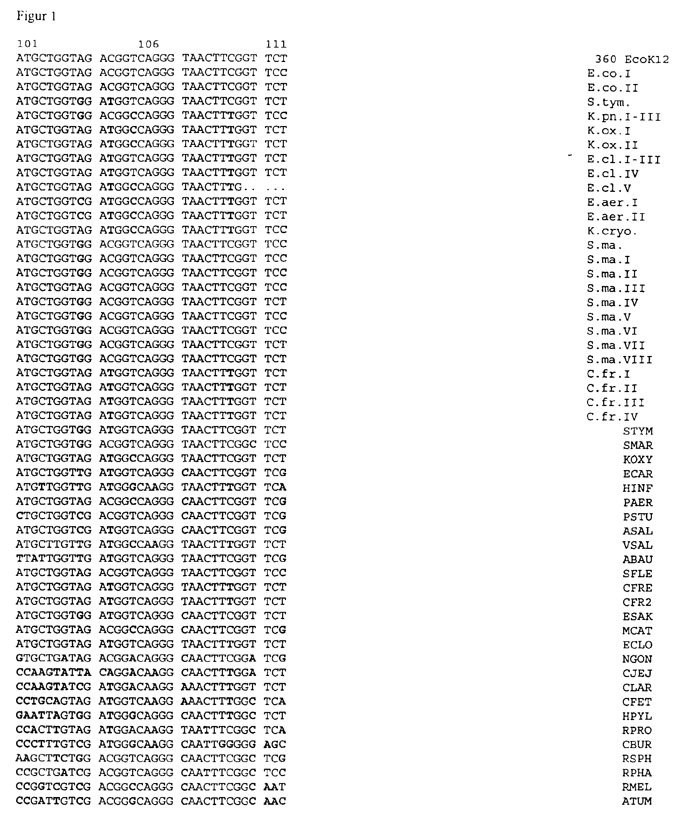

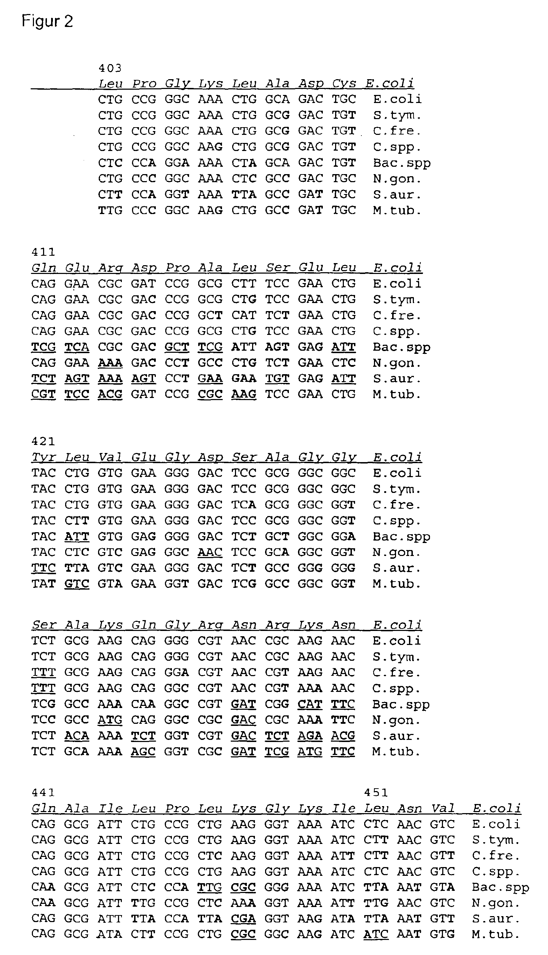

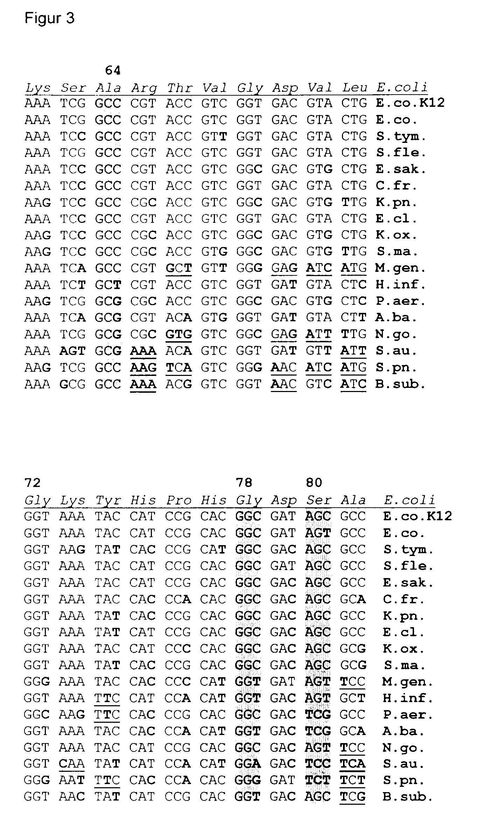

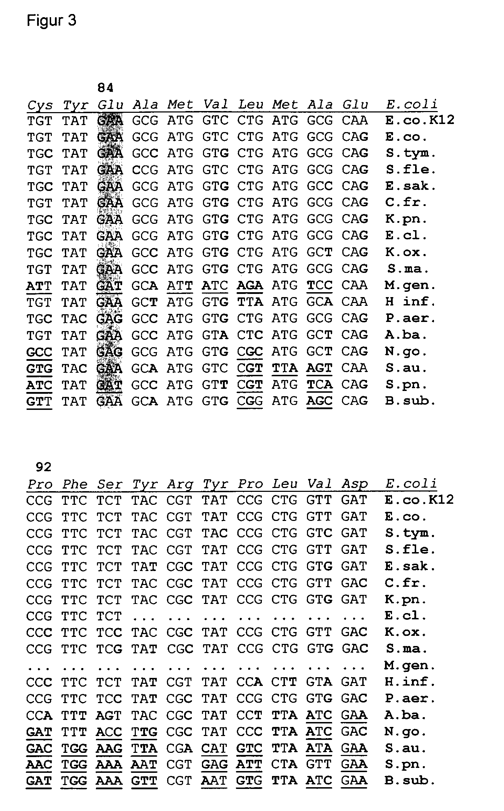

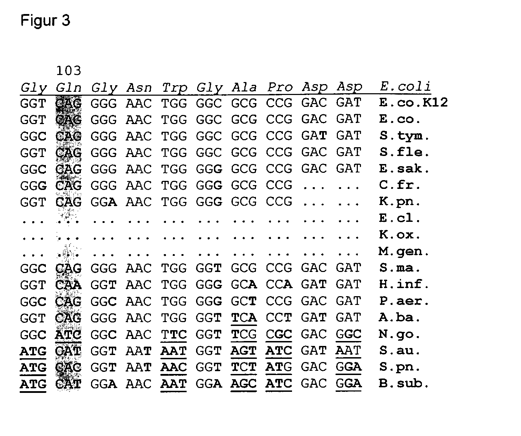

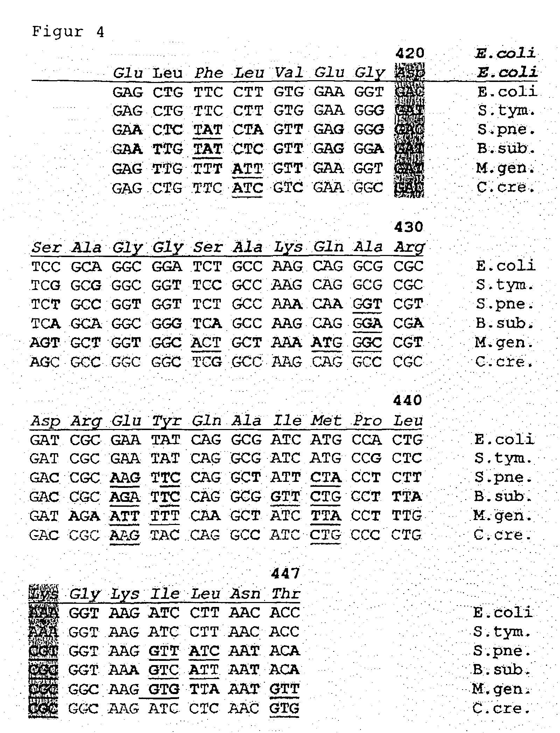

Die Figuren 1 bis 4 zeigen den Vergleich der Sequenzen gyrA, gyrB, parC und parE der verschiedenen Bakterienspezies mit der E.coli K12-Sequenz. Abweichung in den Sequenzen gegenüber der E.coli K12-Sequenz sind jeweils Fett gedruckt. Unterstreichungen kennzeichnen Aminosäureveränderungen (Figuren 2 bis 4).

Das erfindungsgemäße Verfahren soll durch folgendes Beispiel erläutert werden:
Ausgangspunkt für die Untersuchung ist eine repräsentative Einzelkolonie, die aus dem Untersuchungsmaterial (Infektionsherd) eines Patienten isoliert wurde. Diese Kolonie wird in sterilem Wasser (50 bis 100 µl) resuspendiert. Durch Aufkochen (15 min) wird Vorlagen-DNA für eine anschließende Amplifikationsreaktion gewonnen.

Zu dieser Vorlagen-DNA werden spezifische Oligonukleotide als Primerpaare zugesetzt sowie Desoxynukleotidtriphosphate, geeigneter Puffer (z.B. für Taq-DNA-Polymerase für den Fall, daß eine PCR durchgeführt wird) und eine Enzym (z.B. Taq-DNA-Polymerase). Im Falle von Enterobakterien hat sich folgende Kombination von Primern für die Gene gyrA bzw. parC bewährt:

gyrA5-1: 5′-GAATCCGGATACAGTAGAGGGATAG-3′

gyrA3-1: 5′-CCTTAAACCAACCGTACTGCAGGCCT-3′

parC-S: 5′-GTATGCGATGTCTGAACTGGGCCTG-3′

parC-U: 5′-ACCGGGATTCGGTGTAACGCATTGC-3′

parE5′:	5′-GAG CTG TTC CTT GTG GAA GG-3′
oder	5′-GAG CTG TTT CTT GTG GAG GG-3'

parE3′:	5′-GGT GTT AAG GAT CTT ACC-3′
oder	5′-GGT ATT AAG GAC CTT ACC-3′

gyrB5′:    5′-CTG CCG GGC AAA CTG GC-3′
oder    5′-CTG CCG GGC AAA CTA GC-3′

gyrB3′:	5′-AC GTT GAG GAT TTT ACC-3′
oder	5′-AC GTT AAG AAT TTT ACC-3′

Die durch Amplifikation (30 Zyklen mit folgendem Temperaturprofil 30 s 95°C, 30 s 50°C; 45 s 72°C) erhaltenen DNA-Fragmente werden durch Abtrennung von Primer, Enzym und Vorlagen-DNA gereinigt.

Das gereinigte Fragment wird für eine DNA-Sequenzanalyse der QRDR-Bereiche eingesetzt (z.B. mittels Hybridisierung mit einem Array aus Octamer-Oligonukleotiden, SBH). Ziel ist die Identifizierung von spezifischen Sequenzvariationen, wie sie in den Figuren 1 bis 4 als charakteristisch für einzelne Species oder Subspecies gekennzeichnet sind.

Aufgrund der Übereinstimmung dieser ermittelten Sequenzvariationen im Vergleich mit bekannten Variationen (in einer Datenbank) wird dann das untersuchte Isolat einer Species zugeordnet.

SEQUENZPROTOKOLL

<110> Merlin Gesellschaft für mikrobiologische Diagnosti

<120> Verfahren zur genotypischen Klassifizierung

<130> 982216de Merlin

<140>
<141>

<160> 13

<170> PatentIn Ver. 2.1

<210> 1
<211> 120
<212> DNA
<213> Escherichia coli

<400> 1
gcccgtgtcg ttggtgacgt aatcggtaaa taccatcccc atggtgactc ggcggtttat 60
gacacgatcg tccgtatggc gcagccattc tcgctgcgtt acatgctggt agacggtcag 120

<210> 2
<211> 66
<212> DNA
<213> Escherichia coli

<400> 2
gactccgcgg gcggctctgc gaagcagggg cgtaaccgca agaaccaggc gattctgccg 60
ctgaag 66

<210> 3
<211> 120
<212> DNA
<213> Escherichia coli

<400> 3
gcccgtaccg tcggtgacgt actgggtaaa taccatccgc acggcgatag cgcctgttat 60
gaagcgatgg tcctgatggc gcaaccgttc tcttaccgtt atccgctggt tgatggtcag 120

<210> 4
<211> 66
<212> DNA
<213> Escherichia coli

<400> 4

gactccgcag gcggatctgc caagcaggcg cgcgatcgcg aatatcaggc gatcatgcca 60
ctgaaa 66

<210> 5
<211> 180
<212> DNA
<213> Escherichia coli

<400> 5
atgaacgtac taggcaatga ctggaacaaa gcctataaaa aatctgcccg tgtcgttggt 60
gacgtaatcg gtaaatacca tccccatggt gactcggcgg tttatgacac gatcgtccgt 120
atggcgcagc cattctcgct gcgttacatg ctggtagacg gtcagggtaa cttcggttcc 180

<210> 6
<211> 180
<212> DNA
<213> Enterobacter aerogenes

<400> 6
atgaacgtat tgggcaatga ctggaacaaa gcctataaaa aatcagcccg tgtcgttggc 60
gacgtaatcg gtaaatacca cccgcatggt gataccgcgg tttatgacac catcgtacgt 120
atggcgcagc cgttctcctt gcgttatatg ctggtcgatg gccagggtaa ctttggttct 180

<210> 7
<211> 180
<212> DNA
<213> Pseudomonas aeruginosa

<400> 7
atgagcgagc tgggcaacga ctggaacaag ccctacaaga aatccgcccg tgtggtcggc 60
gacgtgatcg gtaagtacca cccgcacggc gacaccgcgg tctacgacac catcgtgcgc 120
atggcgcagc cgttctcgct gcgctacatg ctggtagacg gccagggcaa cttcggttcg 180

<210> 8
<211> 180
<212> DNA
<213> Streptococcus pneumoniae

<400> 8
atgaatgaat tgggtgtgac cccagacaaa ccccataaaa aatctgctcg tattacaggg 60
gatgtcatgg gtaaatacca cccacacggg gattcctcta tttatgaagc catggtccgt 120
atggctcaat ggtggagcta ccgttacatg cttgtagatg gtcatgggaa ttttggttcc 180

<210> 9
<211> 153
<212> DNA
<213> Streptococcus pneumoniae

<400> 9
ctgccgggca aactggcaga ctgccaggaa cgcgatccgg cgctttccga actgtacctg 60
gtggaagggg actccgcggg cggctctgcg aagcaggggc gtaaccgcaa gaaccaggcg 120
attctgccgc tgaagggtaa aatcctcaac gtc 153

<210> 10
<211> 180
<212> DNA
<213> Streptococcus pneumoniae

<400> 10
atgtctgaac tgggcctgaa tgccagcgcc aaatttaaaa aatcggcccg taccgtcggt 60
gacgtactgg gtaaatacca tccgcacggc gatagcgcct gttatgaagc gatggtcctg 120
atggcgcaac cgttctctta ccgttatccg ctggttgatg gtcaggggaa ctggggcgcg 180

<210> 11
<211> 165
<212> DNA
<213> Streptococcus pneumoniae

<400> 11
ctggatgccg attccaagca caagaagtcg gcgcgcaccg tcggcgacgt gctcggcaag 60
ttccacccgc acggcgactc ggcctgctac gaggccatgg tgctgatggc gcagccgttc 120
tcctatcgct atccgctggt ggacggccag ggcaactggg gggct 165

<210> 12
<211> 180
<212> DNA
<213> Streptococcus pneumoniae

<400> 12
atgaataagg atagcaatac ttttgacaag agctaccgta agtcggccaa gtcagtcggg 60
aacatcatgg ggaatttcca cccacacggg gattcttcta tctatgatgc catggttcgt 120
atgtcacaga actggaaaaa tcgtgagatt ctagttgaaa tgcacggtaa taacggttct 180

<210> 13
<211> 197
<212> DNA
<213> Streptococcus pneumoniae

<400> 13
cgttgcctgg caaactggct gattgtaccg cgcaggacct taaccgtacc gagctgttcc 60
ttgtggaagg tgactccgca ggcggatctg ccaagcaggc gcgcgatcgc gaatatcagg 120
cgatcatgcc actgaaaggt aagatcctta acacctggga agtctcttcc gacgaagtgc 180
tggcttcgca ggaagtg 197

### Annex zu den Anmeldungsunterlagen - nachgereichtes Sequenzprotokoll

SEQUENZPROTOKOLL

<110> Merlin Ges. für mikrobiologische Diagnostika mbH

<120> Verfahren zur genotypischen Klassifizierung

<130> 99116340.3

<140> 99116340.3
<141> 1999-08-19

<160> 103

<170> PatentIn Ver. 2.1

<210> 1
<211> 213
<212> DNA
<213> Escherichia coli

<220>
<223> Figur 1, gyrA, partial sequence, EcoK12

<300>

<400> 1
ctgaagccgg tacaccgtcg cgtactttac gccatgaacg tactaggcaa tgactggaac 60
aaagcctata aaaaatctgc ccgtgtcgtt ggtgacgtaa tcggtaaata ccatccccat 120
ggtgactcgg cggtctatga cacgattgtc cgcatggcgc agccattctc gctgcgttat 180
atgctggtag acggtcaggg taacttcggt tct 213


<210> 2
<211> 213
<212> DNA
<213> Aeromonas salmonicida

<220>
<223> Figur 1, gyrA, partial sequence, ASAL

<400> 2
ttgaaaccgg ttcaccgccg cgttctgttt gctatgaacg agttggggaa cgactggaac 60
aagccctata aaaaatcggc ccgtgtggtc ggtgacgtaa ttggtaaata ccacccgcac 120
ggcgacagtg ccgtgtatga caccattgtc cgcttggcgc aggatttctc catgcgttac 180
atgctggtcg atggtcaggg caacttcggt tcg 213


<210> 3
<211> 213
<212> DNA
<213> Agrobacterium tumefaciens

<220>
<223> Figur 1, gyrA, partial sequence, ATUM

<400> 3
ctgaagccgg tgcaccgccg catcattcac gccatgagtg aaatgggtat tcgtcccaac 60
tcggccttca agaaatgcgc gcgtatcgtc ggcgatgtga tcggtaagtt ccacccgcat 120
ggcgaccagt cggtctatga tgcgctggtg cgtctcgcgc aggatttctc gcagcgttat 180
ccgattgtcg acgggcaggg caacttcggc aac 213


<210> 4

<211> 213
<212> DNA
<213> Coxiella burnetii

<220>
<223> Figur 1, gyrA, partial sequence, CBUR

<400> 4
ttaaagcccg tccagcgtcg aatcgtgtac gccatgtcag aattgggttt aaaatcaacc 60
gctaagtata agaaatcagc gcggacggta ggcgacgttt tgggtaaatt ccatccgcac 120
ggagacaccg cctgttacga ggccatggta ttgatggccc aacctttttc atttcgctat 180
ccctttgtcg atgggcaagg caattggggg agc 213

<210> 5
<211> 213
<212> DNA
<213> Campylobacter fetus

<220>
<223> Figur 1, gyrA, partial sequence, CFET

<400> 5
ttaaaaccgg ttcatcgtcg catactttat gctatgaacg atcttggcgt aggtagtcgc 60
agcccatata aaaagtctgc tcgtatagta ggtgatgtta tcggtaagta tcacccgcac 120
ggcgatactg cggtatatga cgctttagtt agaatggctc agaacttttc tatgagagtt 180
cctgcagtag atggtcaagg aaactttggc tca 213

<210> 6
<211> 180
<212> DNA
<213> Citrobacter freundii

<220>
<223> Figur 1, gyrA, partial sequence, C.fr.I

<400> 6
atgaacgtat tgggcaacga ctggaataaa gcctataaaa aatctgcccg tgtcgttggt 60
gacgtaatcg gtaaatacca ccctcatggt gataccgccg tttacgacac cattgttcgt 120
atggcgcagc cattctcctt gcgttacatg ctggtagatg gtcagggtaa ctttggttct 180

<210> 7
<211> 213
<212> DNA
<213> Citrobacter freundii

<220>
<223> Figur 1, gyrA. partial sequence, CFR2

<400> 7
ctgaaaccgg ttcaccgtcg cgtactttac gccatgaacg tattgggcaa cgactggaat 60
aaagcctata aaaaatctgc ccgtgtcgtt ggtgacgtaa tcggtaaata ccaccctcat 120
ggtgataccg ccgtttacga caccattgtt cgtatggcgc agccattctc cttgcgttac 180
atgctggtag atggtcaggg taactttggt tct 213

<210> 8
<211> 180
<212> DNA
<213> Citrobacter freundii

<220>
<223> Figur 1, gyrA, partial sequence, C.fr.III

<400> 8
atgaacgtat tgggcaacga ctggaataaa gcctataaaa aatctgcccg tgtcgttggt 60
gacgtaatcg gtaaatacca ccctcatggt gataccgccg tttacgacac cattgttcgt 120
atggcgcagc cattctcctt gcgctatatg ctggtagatg gtcagggtaa ctttggttct 180


<210> 9
<211> 180
<212> DNA
<213> Citrobacter freundii

<220>
<223> Figur 1, gyrA, partial sequence, C.fr.IV

<400> 9
atgaacgtat tgggcaacga ctggaataaa gcctataaaa aatctgcccg tgtcgttggt 60
gacgtaatcg gtaaatacca ccctcatggt gatactgccg tttacgacac cattgttcgt 120
atggcgcagc cattctcctt gcgttacatg ctggtagatg gtcagggtaa ctttggttct 180


<210> 10
<211> 114
<212> DNA
<213> Citrobacter freundii

<220>
<223> Figur 1, gyrA, partial sequence, CFRE

<400> 10
atcggtaaat accaccctca tggtgatacc gccgtttacg acaccattgt tcgtatggcg 60
cagccattct ccttgtgtta catgctggta gatggtcagg gtaactttgg ttct 114


<210> 11
<211> 213
<212> DNA
<213> Campylobacter jejuni

<220>
<223> Figur 1, gyrA, partial sequence, CJEJ

<400> 11
ttaaagcctg ttcatagaag aattttatat gctatgcaaa atgatgaggc aaaaagtaga 60
acagattttg tcaaatcagc ccgtatagtg ggtgctgtta taggtcgtta tcacccacat 120
ggagatacag cagtttatga tgctttggtt agaatggctc aagatttttc tatgagatat 180
ccaagtatta caggacaagg caactttgga tct 213


<210> 12
<211> 201
<212> DNA
<213> Campylobacter lari

<220>
<223> Figur 1, gyrA, partial sequence, CLAR

<400> 12
cacagaagaa tactttatgc tatgaatgat cttggcgtag gaagtagaag tgcatataaa 60
aaatctgctc gtatagtagg ggatgttatc ggtaagtatc atccacatgg cgatactgct 120
gtttacgatg ccttagtaag aatggcacaa gatttctcta tgcgttatcc aagtatcgat 180

ggacaaggaa actttggttc t 201

<210> 13
<211> 180
<212> DNA
<213> Enterobacter aerogenes

<220>
<223> Figur 1, gyrA, partial sequence, E.aer.I

<400> 13
atgaacgtat tgggcaatga ctggaacaaa gcctataaaa aatcagcccg tgtcgttggc 60
gacgtaatcg gtaaatacca cccgcatggt gataccgcgg tttatgacac catcgtacgt 120
atggcgcagc cgttctcctt gcgttatatg ctggtcgatg gccagggtaa ctttggttct 180

<210> 14
<211> 93
<212> DNA
<213> Enterobacter aerogenes

<220>
<223> Figur 1, gyrA, partial sequence, E.aer.II

<400> 14
ggtgataccg cggtttatga caccatcgta cgtatggcgc agccgttctc tttgcgttat 60
atgctggtcg atggccaggg taactttggt tct 93

<210> 15
<211> 213
<212> DNA
<213> Erwinia carotovora

<220>
<223> Figur 1, gyrA, partial sequence, ECAR

<400> 15
ctgaaaccgg tacaccgccg cgtactgtat gcgatgagcg tactgggtaa cgactggaac 60
aaaccgtata aaaaatccgc ccgtgtcgtc ggggatgtca tcggtaaata ccacccacac 120
ggcgactctg ccgtttatga aaccatcgta cgtatggcgc agcctttctc actgcgttac 180
atgctggttg atggtcaggg caacttcggt tcg 213

<210> 16
<211> 165
<212> DNA
<213> Enterobacter cloacae

<220>
<223> Figur 1, gyrA, partial sequence, E.cl.I

<400> 16
aatgactgga ataaagccta caaaaaatct gcccgtgtcg ttggtgacgt aatcggtaaa 60
taccatcctc atggtgattc cgcggtgtac gacaccattg tccgtatggc gcagcctttc 120
tcgctgcgtt acatgctggt agatggtcag ggtaactttg gttct 165

<210> 17
<211> 165
<212> DNA
<213> Enterobacter cloacae

<220>
<223> Figur 1, gyrA, partial sequence, E.cl.II

<400> 17
aatgactgga ataaagccta taaaaaatct gcccgtgtcg ttggtgacgt aatcggtaaa 60
taccatcctc atggtgattc cgcggtgtac gacaccattg tccgtatggc gcagcctttc 120
tcgctgcgtt acatgctggt agatggtcag ggtaactttg gttct 165


<210> 18
<211> 180
<212> DNA
<213> Enterobacter cloacae

<220>
<223> Figur 1, gyrA, partial sequence, E.cl.III

<400> 18
atgaacgtat tgggcaatga ctggaataaa gcctacaaaa aatctgcccg tgtcgttggt 60
gacgtaatcg gtaaatacca tccccatggt gattccgcgg tgtacgacac catcgttcgt 120
atggcgcagc ctttctcgct gcgttacatg ctggtagatg gtcagggtaa ctttggttct 180


<210> 19
<211> 180
<212> DNA
<213> Enterobacter cloacae

<220>
<223> Figur 1, gyrA, partial sequence, E.cl.IV

<400> 19
atgaacgtat tgggcaatga ctggaataaa gcctacaaaa aatctgcccg tgtcgttggt 60
gacgtaatcg gtaaatacca tccccatggt gattccgcgg tgtacgacac cattgtccgt 120
atggcgcagc cnttctcgct gcgttacatg ctggtagatg gtcagggtaa ctttggttct 180


<210> 20
<211> 175
<212> DNA
<213> Enterobacter cloacae

<220>
<223> Figur 1, gyrA, partial sequence, E.cl.V

<400> 20
atgaacgtat tcggcaatga ctggaataaa gcctacaaaa aatctgcccg tgtcgttggt 60
gacgtaatcg gtaaatacca ccctcatggt gattctgcgg tgtatgacac cattgtacgt 120
atggcgcagc cattctccct gcgttatatg ctggtagatg gccagggtaa ctttg 175


<210> 21
<211> 169
<212> DNA
<213> Enterobacter cloacae

<220>
<223> Figur 1, gyrA, partial sequence, ECLO

<400> 21
ttgggcaatg actggaataa agcctacaaa aaatctgccc gtgtcgttgg tgacgtaatc 60
ggtaaatacc atccccatgg tgattccgcg gtgtacgaca ccatcgttcg tatggcgcag 120

cctttctcgc tgcgttacat gctggtagat ggtcagggta actttggtt 169

<210> 22
<211> 180
<212> DNA
<213> Escherichia coli

<220>
<223> Figur 1, gyrA, partial sequence, E.co.II

<400> 22
atgaacgtac taggcaatga ctggaacaaa gcctataaaa aatctgcccg tgtcgttggt 60
gacgtaatcg gtaaatacca tccccatggt gactcggcgg tctatgacac gatcgtccgc 120
atggcgcagc cattctcgct gcgttatatg ctggtagacg gtcagggtaa cttcggttct 180

<210> 23
<211> 213
<212> DNA
<213> Enterobacter sakazakii

<220>
<223> Figur 1, gyrA, partial sequence, ESAK

<400> 23
ctcaaaccgg tacaccgtcg cgtactttac gccatgaacg tgttgggcaa tgactggaat 60
aaagcctaca aaaaatccgc ccgtgtcgtt ggtgacgtaa tcggtaaata ccatccccac 120
ggtgattccg ccgtctacga taccattgta cgtatggctc agccgttctc gctgcgctat 180
atgctggtgg atggtcaggg caacttcggt tct 213

<210> 24
<211> 213
<212> DNA
<213> Haemophilus influenzae

<220>
<223> Figur 1, gyrA, partial sequence, HINF

<400> 24
ttaaaaccag ttcaccgccg cgtactgttc tcaatggatc gcgaaggcaa taccgccaat 60
aaaaaatacg taaaatcagc gcgtgttgtg ggtgatgtaa tcggtaaata tcacccgcat 120
ggtgacttag ccgtgtacta taccatcgtt cgtatggcac aaccattctc acttcgctat 180
atgttggttg atgggcaagg taactttggt tca 213

<210> 25
<211> 213
<212> DNA
<213> Helicobacter pylori

<220>
<223> Figur 1, gyrA, partial sequence, HPYL

<400> 25
ttaaagcccg tgcataggcg tattttgtat gcgatgcatg aattaggtct tacttccaaa 60
gtcgcttata aaaaaagcgc taggatcgtg ggtgatgtga ttggtaaata ccacccccat 120
ggcgataacg cggtttatga tgcactagtg agaatggcgc aagatttttc catgcgcttg 180
gaattagtgg atgggcaggg caactttggc tct 213

<210> 26

<211> 180
<212> DNA
<213> Kluyvera cryocrescens

<220>
<223> Figur 1, gyrA, partial sequence, K.cryo

<400> 26
atgaacgtat tgggcaatga ctggaacaaa gcctacaaaa aatcagcccg tgtcgttggt 60
gacgtaatcg gtaaatacca tccccatggt gattccgcgg tgtacgacac catcgtacgt 120
atggcgcagc cnttctcgct gcgttacatg ctggtagatg gccagggtaa ctttggttcc 180


<210> 27
<211> 180
<212> DNA
<213> Klebsiella oxytoca

<220>
<223> Figur 1, gyrA, partial sequence, K.ox.I

<400> 27
atgaacgtat tgggcaatga ctggaacaaa gcctataaaa aatctgcccg tgtcgttggt 60
gacgtaatcg gtaaatacca ccctcatggt gatactgccg tgtatgacac cattgtacgt 120
atggcgcagc cattctccct gcgttacatg ctggtagatg gccagggtaa ctttggttct 180


<210> 28
<211> 180
<212> DNA
<213> Klebsiella oxytoca

<220>
<223> Figur 1, gyrA, partial sequence, K.ox.II

<400> 28
atgaacgtat tgggcaatga ctggaacaaa gcctataaaa aatctgcccg tgtcgtgggt 60
gacgtcatcg gtaaatacca ccctcatggt gatactgccg tatacgacac cattgtacgt 120
atggcgcagc cattctccct gcgttacatg ctggtagatg gccagggtaa ctttggttct 180


<210> 29
<211> 213
<212> DNA
<213> Klebsiella oxytoca

<220>
<223> Figur 1, gyrA, partial sequence, KOXY

<400> 29
ctgaagccgg tacaccgtcg cgtactatac gccatgaacg tattgggcaa tgactggaac 60
aaagcctata aaaaatctgc ccgtgtcgtg ggtgacgtca tcggtaaata ccaccctcat 120
ggtgatactg ccgtatacga caccattgta cgtatggcgc agccattctc cctgcgttac 180
atgctggtag atggccaggg taactttggt tcg 213


<210> 30
<211> 180
<212> DNA
<213> Klebsiella pneumoniae

<220>
<223> Figur 1, gyrA, partial sequence, K.pn.I

<400> 30
atgaacgtat tgggcaatga ctggaacaaa gcctataaaa aatcagcccg tgtcgttggt 60
gacgtaatcg gtaaatacca cccgcacggc gactccgcgg tatacgacac catcgtgcgt 120
atggcgcagc cgttctcgct gcgttacatg ctggtggacg gccagggtaa ctttggttcc 180

<210> 31
<211> 180
<212> DNA
<213> Klebsiella pneumoniae

<220>
<223> Figur 1, gyrA, partial sequence, K.pn.II

<400> 31
atgaacgtat tgggcaatga ctggaacaaa gcctataaaa aatcagcccg tgtcgttggt 60
gacgtaatcg gtaaatacca cccgcacggc gactccgcgg tatacgacac catcgtccgt 120
atggcgcagc cgttctcgct gcgttacatg ctggtggacg gccagggtaa ctttggttcc 180

<210> 32
<211> 180
<212> DNA
<213> Klebsiella pneumoniae

<220>
<223> Figur 1, gyrA, partial sequence, K.pn.III

<400> 32
atgaacgtat tgggcaatga ctggaacaaa gcctataaaa aatcagcccg tgtcgttggt 60
gacgtaatcg gtaaatacca cccgcacggc gactccgcgg tatacgacac catcgttcgt 120
atggcgcagc cgttctcgct gcgttacatg ctggtggacg gccagggtaa ctttggttcc 180

<210> 33
<211> 162
<212> DNA
<213> Moraxella catarrhalis

<220>
<223> Figur 1, gyrA, partial sequence, MCAT

<400> 33
aactggaaca agccctacaa gaaatccgcc cgtgtggtcg gcgacgtgat cggtaagtac 60
cacccgcacg gcgacatcgc ggtctacgac accatcgtgc gcatggcgca gccgttctcg 120
ctgcgctaca tgctggtaga cggccagggc aacttcggtt cg 162

<210> 34
<211> 213
<212> DNA
<213> Neisseria gonorrhoeae

<220>
<223> Figur 1, gyrA, partial sequence, NGON

<400> 34
ctaaagccgg tgcaccggcg cgtactgtac gcgatgcacg agctgaaaaa taactggaat 60
gccgcctaca aaaaatcggc gcgcatcgtc ggcgacgtca tcggtaaata ccacccccac 120
ggcgattccg cagtttacga caccatcgtc cgtatggcgc aaaatttcgc tatgcgttat 180
gtgctgatag acggacaggg caacttcgga tcg 213

<210> 35
<211> 213
<212> DNA
<213> Pseudomonas aeruginosa

<220>
<223> Figur 1, gyrA, partial sequence, PAER

<400> 35
ctgaagccgg tgcaccgccg tgtgctttat gccatgagcg agctgggcaa cgactggaac 60
aagccctaca agaaatccgc ccgtgtggtc ggcgacgtga tcggtaagta ccacccgcac 120
ggcgacaccg cggtctacga caccatcgtg cgcatggcgc agccgttctc gctgcgctac 180
atgctggtag acggccaggg caacttcggt tcg 213

<210> 36
<211> 213
<212> DNA
<213> Providencia stuartii

<220>
<223> Figur 1, gyrA, partial sequence, PSTU

<400> 36
ttgaaacccg tgcaccgccg tgtgctgttt gccatgagcg aactgggtaa cgactggaac 60
aagccgtaca agaaatcggc ccgtgtcgtg ggtgacgtga tcggtaaata ccacccccac 120
ggcgactcgg cggtttacga caccatcgtc cgcatggccc agccattctc gctgcgatac 180
ctgctggtcg acggtcaggg caacttcggt tcg 213

<210> 37
<211> 213
<212> DNA
<213> Rhizobium phaseoli

<220>
<223> Figur 1, gyrA, partial sequence, RPHA

<400> 37
ctgaagccgg tgcaccggcg catcctctac ggcatgtccg agctcggtat cgactggaac 60
aaaaaatacg tcaaatgcgc ccgcgtcacc ggcgacgtga tgggtaaata tcatccgcac 120
ggcaatgccg cgatctatga tgcgctcgcc cgcatggcgc agccctggtc gctgcggctg 180
ccgctgatcg acggtcaggg caatttcggc tcc 213

<210> 38
<211> 213
<212> DNA
<213> Rickettsia prowazekii

<220>
<223> Figur 1, gyrA, partial sequence, RPRO

<400> 38
ttaaagcctg tgcatcgccg aattatctat tccatgtatg aagccggtaa tcatgctagc 60
aaaccttata gaaaatctgc acgaatagtt ggtgacgtga tgggtaaata tcatcctcac 120
ggtgatagtg ctatttatga ctcgttagta cgtatggctc aagatttttc tttgcgtcta 180
ccacttgtag atggacaagg taatttcggc tca 213

<210> 39
<211> 213

<212> DNA
<213> Rhodobacter sphaeroides

<220>
<223> Figur 1, gyrA, partial sequence, RSPH

<400> 39
ctgaagccgg tgcaccgccg catcctctat gcgatgcacg agacggggaa cacgcacgac 60
aagccctacc gcaagtcggc gcgcccggtg ggcgacacga tggggaaata ccacccccac 120
ggcgatggcg cgatctatga cgcgctggtg cggatggcgc agcccttctc gatggggctg 180
aagcttctgg acggtcaggg caacttcggc tcg 213


<210> 40
<211> 168
<212> DNA
<213> Shigella flexneri

<220>
<223> Figur 1, gyrA, partial sequence, SFLE

<400> 40
ggcaatgact ggaacaaagc ctataaaaaa tctgcccgtg ccgttggtga cgtaatcggt 60
aaataccatc cccatggtga ctcggcggtt tatgacacga tcgtccgtat ggcgcagcca 120
ttctcgctgc gttacatgct ggtagacggt cagggtaact tcggttcc 168


<210> 41
<211> 180
<212> DNA
<213> Serratia marcescens

<220>
<223> Figur 1, gyrA, partial sequence, S.ma.I

<400> 41
atgagtgtat tgggtaacga ctggaataaa ccatacaaga aatcggcccg tgtcgtcggg 60
gacgtgatcg gtaaatatca cccgcacggt gacagcgcgg tttacgacac tatcgtgcgt 120
atggctcagc cgttttcact gcgctacatg ctggtggacg gtcagggtaa cttcggttcc 180


<210> 42
<211> 180
<212> DNA
<213> Serratia marcescens

<220>
<223> Figur 1, gyrA, partial sequence, S.ma.II

<400> 42
atgagcgtat tgggtaacga ctggaataaa ccatacaaga aatcggcccg tgtcgtcggg 60
gatgtgatcg gtaaatatca cccacacggt gacagcgcgg tttacgacac tatcgtgcgt 120
atggctcagc cgttttcact gcgctacatg ctggtggacg gtcagggtaa cttcggttcc 180


<210> 43
<211> 180
<212> DNA
<213> Serratia marcescens

<220>
<223> Figur 1, gyrA, partial sequence, S.ma.III

<400> 43
atgaacgtat tgggtaacga ctggaataaa ccatacaaga aatcggcccg tgtcgtcggg 60
gacgtaatcg gtaaatacca ccctcatggt gacagcgcgg tgtacgacac gatcgtgcgt 120
atggcgcagc cnttntcnct gcgttacatg ctggtagacg gtcagggtaa cttcggttcc 180

<210> 44
<211> 180
<212> DNA
<213> Serratia marcescens

<220>
<223> Figur 1, gyrA, partial sequence, S.ma.IV

<400> 44
atgaacgtat taggtaacga ctggaataaa ccatacaaga aatcggcccg tgtcgtcggg 60
gacgtaatcg gtaaatatca cccgcacggt gacagcgccg tttacgacac tatcgtgcgt 120
atggctcagc cgttttcact gcgctacatg ctggtggacg gtcagggtaa cttcggttct 180

<210> 45
<211> 180
<212> DNA
<213> Serratia marcescens

<220>
<223> Figur 1, gyrA, partial sequence, S.ma.V

<400> 45
atgagtgtat tgggtaacga ctggaataaa ccatataaga aatctgcccg tgtcgttggg 60
gacgtaatcg gtaaatatca cccgcacggt gacagcgccg tttacgacac tatcgtgcgt 120
atggctcagc cgttttcact gcgctacatg ctggtggacg gtcagggtaa cttcggttcc 180

<210> 46
<211> 180
<212> DNA
<213> Serratia marcescens

<220>
<223> Figur 1, gyrA, partial sequence, S.ma.VI

<400> 46
atganngtat tgggnaacga ctggaataaa ccatacaaaa aatcggcccg tgtcgtcggg 60
gacgtgatcg gtaaatatca ccctcacggt gacagcgccg tntacgacac tatcgtncgt 120
atggctcagc cgttttcact gcgttacatg ctggtggacg gtcagggtaa cttcggttcc 180

<210> 47
<211> 180
<212> DNA
<213> Serratia marcescens

<220>
<223> Figur 1, gyrA, partial sequence, S.ma.VII

<400> 47
atgagtgtac tgggtaacga ctggaataaa ccatacaaga aatcggcccg tgtcgtcggg 60
gacgtaatcg gtaaatatca cccacacggt gacagcgcgg tttacgacac tatcgtgcgt 120
atggcccagc cgttttcact gcgttacatg ctggtggacg gtcagggtaa cttcggttct 180

<210> 48

<211> 180
<212> DNA
<213> Serratia marcescens

<220>
<223> Figur 1, gyrA, partial sequence, S.ma.VIII

<400> 48
atgagcgtat tgggtaacga ctggaataaa ccatacaaga agtcggcccg tgtcgtcggg 60
gacgtgatcg gtaaatatca cccgcacggt gacagcgcgg tttacgacac tatcgtgcgt 120
atggctcagc cgttttcact gcgctacatg ctggtggacg gtcagggtaa cttcggttct 180


<210> 49
<211> 213
<212> DNA
<213> Serratia marcescens

<220>
<223> Figur 1, gyrA, partial sequence, SMAR

<400> 49
ctgaagccgg ttcaccgccg cgttctgtac gcgatgagcg tattgggtaa cgactggaat 60
aaaccataca agaaatcggc ccgtgtcgtc ggggacgtga tcggtaaata tcacccgcac 120
ggtgacagcg cggtttacga cactatcgtg cgtatggctc agccgttttc actgcgctac 180
atgctggtgg acggtcaggg taacttcggc tcc 213


<210> 50
<211> 180 .
<212> DNA
<213> Salmonella typhimurium

<220>
<223> Figur 1, gyrA, partial sequence, S.ty.

<400> 50
atgaacgtat tgggcaatga ctggaacaaa gcctataaaa aatctgcccg tgtcgttggt 60
gacgtaatcg gtaaatacca tccccacggc gattccgcag tgtatgacac catcgttcgt 120
atggcgcagc cattctcgct gcgttacatg ctggtggatg gtcagggtaa cttcggttct 180


<210> 51
<211> 147
<212> DNA
<213> Salmonella typhimurium

<220>
<223> Figur 1, gyrA, partial sequence, STYM

<400> 51
tataaaaaat ctgcccgtgt cgttggtgac gtaatcggta aataccatcc ccacggcgat 60
tccgcagtgt atgacaccat cgttcgtatg gcgcagccat tctcgctgcg ttacatgctg 120
gtggatggtc agggtaactt cggttct 147


<210> 52
<211> 213
<212> DNA
<213> Vibrio salmonicida

<220>
<223> Figur 1, gyrA, partial sequence, VSAL

<400> 52
ctaaaacctg tacaccgccg tgttttattc gcgatggatg tattaggtaa tgattggaat 60
aaaccatata aaaaatctgc ccgtgtcgtc ggcgacgtaa ttggtaagta tcacccacat 120
ggtgatagtg ctgtatacga cacgatagta cgtattgcgc agccgttctc actacgctat 180
atgcttgttg atggccaagg taactttggt tct 213

<210> 53
<211> 126
<212> DNA
<213> Acinetobacter baumannii

<220>
<223> Figur 1, gyrA, partial sequence, ABAU

<400> 53
gttggtgacg taatcggtaa atatcacccg catggtgact cagctgttta tgaaaccatt 60
gttcgtatgg ctcaagactt tagcttacgt tatttattgg ttgatggtca gggtaacttc 120
ggttcg 126

<210> 54
<211> 180
<212> DNA
<213> Escherichia coli

<220>
<223> Figur 1, gyrA, partial sequence, E.co.I

<400> 54
atgaacgtac taggcaatga ctggaacaaa gcctataaaa aatctgcccg tgtcgttggt 60
gacgtaatcg gtaaatacca tccccatggt gactcggcgg tttatgacac gatcgtccgt 120
atggcgcagc cattctcgct gcgttacatg ctggtagacg gtcagggtaa cttcggttcc 180

<210> 55
<211> 213
<212> DNA
<213> Sinorhizobium meliloti

<220>
<223> Figur 1, gyrA, partial sequence, RMEL

<400> 55
ctgaagccgg tgcatcgccg gatcattcat gccatgagcg agatgggcct caggcccaat 60
tcctcgttca agaaatgcgc ccgtatcgtc ggcgacgtca tcggtaagtt ccacccgcat 120
ggcgaccagt cggtctatga cgcgctggta cgcctcgcgc aggacttctc ccagcgctat 180
ccggtcgtcg acggccaggg caacttcggc aat 213

<210> 56
<211> 153
<212> DNA
<213> Bacillus spp.

<220>
<223> Figur 2, gyrB, partial sequence, Bac.spp

<400> 56
ctcccaggaa aactagcaga ctgttcgtca cgcgacgctt cgattagtga gatttacatt 60
gtggaggggg actctgctgg cggatcggcc aaacaaggcc gtgatcggca tttccaagcg 120
attctcccat tgcgcgggaa aatcttaaat gta 153

<210> 57
<211> 153
<212> DNA
<213> Citrobacter freundii

<220>
<223> Figur 2, gyrB, partial sequence, C.fre.

<400> 57
ctgccgggca aactggcgga ctgtcaggaa cgcgacccgg ctcattctga actgtacctg 60
gtggaagggg actcagcggg cggttttgcg aagcagggac gtaaccgtaa gaaccaggcg 120
attctgccgc tcaagggtaa aattcttaac gtt 153

<210> 58
<211> 153
<212> DNA
<213> Citrobacter spp.

<220>
<223> Figur 2, gyrB, partial sequence, C.spp.

<400> 58
ctgccgggca agctggcgga ctgtcaggaa cgcgacccgg cgctgtccga actgtacctt 60
gtggaagggg actccgcggg cggttttgcg aagcagggcc gtaaccgtaa aaaccaggcg 120
attctgccgc tgaagggtaa aatcctcaac gtc 153

<210> 59
<211> 153
<212> DNA
<213> Escherichia coli

<220>
<221> CDS
<222> (1)..(153)
<223> Figur 2, gyrB, partial sequence, E.coli

<400> 59
ctg ccg ggc aaa ctg gca gac tgc cag gaa cgc gat ccg gcg ctt tcc 48
Leu Pro Gly Lys Leu Ala Asp Cys Gln Glu Arg Asp Pro Ala Leu Ser
1               5                   10                  15

gaa ctg tac ctg gtg gaa ggg gac tcc gcg ggc ggc tct gcg aag cag 96
Glu Leu Tyr Leu Val Glu Gly Asp Ser Ala Gly Gly Ser Ala Lys Gln
            20                  25                  30

ggg cgt aac cgc aag aac cag gcg att ctg ccg ctg aag ggt aaa atc 144
Gly Arg Asn Arg Lys Asn Gln Ala Ile Leu Pro Leu Lys Gly Lys Ile
        35                  40                  45

ctc aac gtc 153
Leu Asn Val
    50

<210> 60
<211> 51
<212> PRT
<213> Escherichia coli

<400> 60
Leu Pro Gly Lys Leu Ala Asp Cys Gln Glu Arg Asp Pro Ala Leu Ser
1               5                   10                  15

Glu Leu Tyr Leu Val Glu Gly Asp Ser Ala Gly Gly Ser Ala Lys Gln
            20                  25                  30

Gly Arg Asn Arg Lys Asn Gln Ala Ile Leu Pro Leu Lys Gly Lys Ile
        35                  40                  45

Leu Asn Val
    50

<210> 61
<211> 153
<212> DNA
<213> Mycobacterium tuberculosis

<220>
<223> Figur 2, gyrB, partial sequence, M.tub.

<400> 61
ttgcccggca agctggccga ttgccgttcc acggatccgc gcaagtccga actgtatgtc 60
gtagaaggtg actcggccgg cggttctgca aaaagcggtc gcgattcgat gttccaggcg 120
atacttccgc tgcgcggcaa gatcatcaat gtg                              153

<210> 62
<211> 153
<212> DNA
<213> Neisseria gonorrhoeae

<220>
<223> Figur 2, gyrB, partial sequence, N.gon.

<400> 62
ctgcccggca aactcgccga ctgccaggaa aaagaccctg ccctgtctga actctacctc 60
gtcgagggca actccgcagg cggttccgcc atgcagggcc gcgaccgcaa attccaagcg 120
attttgccgc tcaaaggtaa aattttgaac gtc                              153

<210> 63
<211> 153
<212> DNA
<213> Staphylococcus aureus

<220>
<223> Figur 2, gyrB, partial sequence, S.aur.

<400> 63
cttccaggta aattagccga ttgctctagt aaaagtcctg aagaatgtga gattttctta 60
gtcgaagggg actctgccgg ggggtctaca aaatctggtc gtgactctag aacgcaggcg 120
attttaccat tacgaggtaa gatattaaat gtt                              153

<210> 64
<211> 153
<212> DNA
<213> Salmonella typhimurium

<220>

<223> Figur 2, gyrB, partial sequence, S.tym.

<400> 64
ctgccgggca aactggcgga ctgtcaggaa cgcgacccgg cgctgtccga actgtacctg 60
gtggaagggg actccgcggg cggctctgcg aagcaggggc gtaaccgcaa gaaccaggcg 120
attctgccgc tgaagggtaa aatccttaac gtc 153

<210> 65
<211> 150
<212> DNA
<213> Acinetobacter baumannii

<220>
<223> Figur 3, parC, partial sequence, A.ba.

<400> 65
aaatcagcgc gtacagtggg tgatgtactt ggtaaatacc acccacatgg tgactcggca 60
tgttatgaag ccatggtact catggctcag ccatttagtt accgctatcc tttaatcgaa 120
ggtcagggga actggggttc acctgatgat 150

<210> 66
<211> 150
<212> DNA
<213> Bacillus subtilis

<220>
<223> Figur 3, parC, partial sequence, B. sub.

<400> 66
aaagcggcca aaacggtcgg taacgtcatc ggtaactatc atccgcacgg tgacagctcg 60
gtttatgaag caatggtgcg gatgagccag gattggaaag ttcgtaatgt gttaatcgaa 120
atgcatggaa acaatggaag catcgacgga 150

<210> 67
<211> 144
<212> DNA
<213> Citrobacter freundii

<220>
<223> Figur 3, parC, partial sequence, C.fr.

<400> 67
   aaatccgccc gtaccgtcgg tgacgtactg ggtaaatacc acccacacgg cgacagcgca 60
   tgttatgaag cgatggtgct gatggcgcag ccgttctctt accgctatcc gctggttgac 120
   gggcagggga actgggggcg gccg 144

<210> 68
<211> 99
<212> DNA
<213> Enterobacter cloacae

<220>
<223> Figur 3, parC, partial sequence, E.cl.

<400> 68
aaatccgccc gtaccgtcgg tgacgtactg ggtaaatatc atccgcacgg cgacagcgcc 60
tgctatgaag cgatggtgct gatggcgcag ccgttctct 99

<210> 69
<211> 150
<212> DNA
<213> Escherichia coli

<220>
<223> Figur 3, parC, partial sequence, E.co.

<400> 69
aaatcggccc gtaccgtcgg tgacgtactg ggtaaatacc atccgcacgg cgatagtgcc 60
tgttatgaag cgatggtcct gatggcgcag ccgttctctt accgttatcc gctggttgat 120
ggtcagggga actggggcgc gccggacgat 150

<210> 70
<211> 150
<212> DNA
<213> Escherichia coli

<220>
<221> CDS
<222> (1)..(150)
<223> Figur 3, parC, partial sequence, E.co.K12

<400> 70
aaa tcg gcc cgt acc gtc ggt gac gta ctg ggt aaa tac cat ccg cac 48
Lys Ser Ala Arg Thr Val Gly Asp Val Leu Gly Lys Tyr His Pro His
1               5                   10                  15

ggc gat agc gcc tgt tat gaa gcg atg gtc ctg atg gcg caa ccg ttc 96
Gly Asp Ser Ala Cys Tyr Glu Ala Met Val Leu Met Ala Gln Pro Phe
            20                  25                  30

tct tac cgt tat ccg ctg gtt gat ggt cag ggg aac tgg ggc gcg ccg 144
Ser Tyr Arg Tyr Pro Leu Val Asp Gly Gln Gly Asn Trp Gly Ala Pro
        35                  40                  45

gac gat 150
Asp Asp
    50

<210> 71
<211> 50
<212> PRT
<213> Escherichia coli

<400> 71
Lys Ser Ala Arg Thr Val Gly Asp Val Leu Gly Lys Tyr His Pro His
1               5                   10                  15

Gly Asp Ser Ala Cys Tyr Glu Ala Met Val Leu Met Ala Gln Pro Phe
            20                  25                  30

Ser Tyr Arg Tyr Pro Leu Val Asp Gly Gln Gly Asn Trp Gly Ala Pro
        35                  40                  45

Asp Asp
    50

<210> 72

<211> 150
<212> DNA
<213> Enterobacter sakazakii

<220>
<223> Figur 3, parC, partial sequence, E.sak.

<400> 72
aaatccgccc gtaccgtcgg cgacgtgctg ggtaaatacc atccgcacgg cgacagcgcc 60
tgctatgaag cgatggtgct gatggcccag ccgttctctt atcgctatcc gctggtggat 120
ggccagggga actggggggc gccggacgat 150


<210> 73
<211> 150
<212> DNA
<213> Haemophilus influenzae

<220>
<223> Figur 3, parC, partial sequence, H.inf.

<400> 73
aaatctgctc gtaccgtcgg tgatgtactc ggtaaattcc atccacatgg tgacagtgct 60
tgttatgaag ctatggtgtt aatggcacaa cccttctctt atcgttatcc acttgtagat 120
ggtcaaggta actggggggc accagatgat 150


<210> 74
<211> 120
<212> DNA
<213> Klebsiella oxytoca

<220>
<223> Figur 3, parC, partial sequence, K.ox.

<400> 74
aagtccgccc gcaccgtcgg cgacgtgctg ggtaaatacc atccccacgg cgacagcgcg 60
tgctatgaag ccatggtgct gatggctcag cccttctcct accgctatcc gctggttgac 120


<210> 75
<211> 144
<212> DNA
<213> Klebsiella pneumoniae

<220>
<223> Figur 3, parC, partial sequence, K.pn.

<400> 75
aagtccgccc gcaccgtcgg cgacgtgttg ggtaaatatc acccgcacgg cgacagcgcc 60
tgctatgaag cgatggtgct gatggcgcag ccgttctctt accgctatcc gctggtggat 120
ggtcagggaa actgggggc gccg 144


<210> 76
<211> 90
<212> DNA
<213> Mycoplasma genitalium

<220>
<223> Figur 3, parC, partial sequence, M.gen.

<400> 76

aaatcagccc gtgctgttgg ggagatcatg gggaaatacc acccccatgg tgatagttcc 60
atttatgatg caattatcag aatgtcccaa 90

<210> 77
<211> 150
<212> DNA
<213> Neisseria gonorrhoeae

<220>
<223> Figur 3, parC, partial sequence, N.go.

<400> 77
aaatcggcgc gcgtggtcgg cgagattttg ggtaaatacc atccgcacgg cgacacttcc 60
gcctatgagg cgatggtgcg catggctcag gattttacct tgcgctatcc cttaatcgac 120
ggcatcggca acttcggttc gcgcgacggc 150

<210> 78
<211> 150
<212> DNA
<213> Pseudomonas aeruginosa

<220>
<223> Figur 3, parC, partial sequence, P.aer.

<400> 78
aagtcggcgc gcaccgtcgg cgacgtgctc ggcaagttcc acccgcacgg cgactcggcc 60
tgctacgagg ccatggtgct gatggcgcag ccgttctcct atcgctatcc gctggtggac 120
ggccagggca actggggggc tccggacgat 150

<210> 79
<211> 150
<212> DNA
<213> Staphylococcus aureus

<220>
<223> Figur 3, parC, partial sequence, S.au.

<400> 79
aaaagtgcga aaacagtcgg tgatgttatt ggtcaatatc atccacatgg agactcctca 60
gtgtacgaag caatggtccg tttaagtcaa gactggaagt tacgacatgt cttaatagaa 120
atgcatggta ataatggtag tatcgataat 150

<210> 80
<211> 150
<212> DNA
<213> Shigella flexneri

<220>
<223> Figur 3, parC, partial sequence, S.fle.

<400> 80
aaatcggccc gtaccgtcgg tgacgtactg ggtaaatacc atccgcacgg cgatagcgcc 60
tgttatgaac cgatggtcct gatggcgcag ccgttctctt accgttatcc gctggttgat 120
ggtcagggga actggggcgc gccggacgat 150

<210> 81
<211> 150
<212> DNA

<213> Serratia marcescens

<220>
<223> Figur 3, parC, partial sequence, S.ma.

<400> 81
aaatccgccc gtaccgttgg tgacgtactg ggtaagtatc acccgcatgg cgacagcgcc 60
tgctatgaag ccatggtgct gatggcgcag ccgttctctt accgttaccc gctggtcgat 120
ggccagggga actggggcgc gccggatgat 150

<210> 82
<211> 150
<212> DNA
<213> Streptococcus pneumoniae

<220>
<223> Figur 3, parC, partial sequence, S.pn.

<400> 82
aagtcggcca agtcagtcgg gaacatcatg gggaatttcc acccacacgg ggattcttct 60
atctatgatg ccatggttcg tatgtcacag aactggaaaa atcgtgagat tctagttgaa 120
atgcacggta ataacggttc tatggacgga 150

<210> 83
<211> 150
<212> DNA
<213> Salmonella typhimurium

<220>
<223> Figur 3, parC, partial sequence, S.tym.

<400> 83
aaatccgccc gtaccgttgg tgacgtactg ggtaagtatc acccgcatgg cgacagcgcc 60
tgctatgaag ccatggtgct gatggcgcag ccgttctctt accgttaccc gctggtcgat 120
ggccagggga actggggcgc gccggatgat 150

<210> 84
<211> 105
<212> DNA
<213> Bacillus subtilis

<220>
<223> Figur 4, parE, partial sequence, B.sub.

<400> 84
gaattgtatc tcgttgaggg agattcagca ggcgggtcag ccaagcaggg acgagaccgc 60
agattccagg cggttctgcc tttacgcggt aaagtcatta ataca 105

<210> 85
<211> 105
<212> DNA
<213> Caulobacter crescentus

<220>
<223> Figur 4, parE, partial sequence, C.cre.

<400> 85
gagctgttca tcgtcgaagg cgacagcgcc ggcggctcgg ccaagcaggc ccgcgaccgc 60
aagtaccagg ccatcctgcc cctgcgcggc aagatcctca acgtg 105

<210> 86
<211> 105
<212> DNA
<213> Escherichia coli

<220>
<221> CDS
<222> (1)..(105)
<223> Figur 4, parE, partial sequence, E.coli

<400> 86
gag ctg ttc ctt gtg gaa ggt gac tcc gca ggc gga tct gcc aag cag 48
Glu Leu Phe Leu Val Glu Gly Asp Ser Ala Gly Gly Ser Ala Lys Gln
1               5                   10                  15

gcg cgc gat cgc gaa tat cag gcg atc atg cca ctg aaa ggt aag atc 96
Ala Arg Asp Arg Glu Tyr Gln Ala Ile Met Pro Leu Lys Gly Lys Ile
            20                  25                  30

ctt aac acc 105
Leu Asn Thr
        35

<210> 87
<211> 35
<212> PRT
<213> Escherichia coli

<400> 87
Glu Leu Phe Leu Val Glu Gly Asp Ser Ala Gly Gly Ser Ala Lys Gln
1               5                   10                  15

Ala Arg Asp Arg Glu Tyr Gln Ala Ile Met Pro Leu Lys Gly Lys Ile
            20                  25                  30

Leu Asn Thr
        35

<210> 88
<211> 105
<212> DNA
<213> Mycoplasma genitalium

<220>
<223> Figur 4, parE, partial sequence, M.gen.

<400> 88
gagttgttta ttgttgaagg tgatagtgct ggtggcactg ctaaaatggg ccgtgataga 60
atttttcaag ctatcttacc tttgcgcggc aaggtgttaa atgtt 105

<210> 89
<211> 105
<212> DNA
<213> Streptococcus pneumoniae

<220>
<223> Figur 4, parE, partial sequence, S.pne.

<400> 89
gaactctatc tagttgaggg ggactctgcc ggtggttctg ccaaacaagg tcgtgaccgc 60
aagttccagg ctattctacc tcttcgtggt aaggttatca ataca 105

<210> 90
<211> 105
<212> DNA
<213> Salmonella typhimurium

<220>
<223> Figur 4, parE, partial sequence, S.tym.

<400> 90
gagctgttcc ttgtggaagg ggattcggcg ggcggttccg ccaagcaggc gcgcgatcgc 60
gaatatcagg cgatcatgcc gctcaaaggt aagatcctta acacc 105

<210> 91
<211> 180
<212> DNA
<213> Citrobacter freundii

<220>
<223> Figur 1, gyrA, partial sequence, C.fr.II

<400> 91
atgaacgtat tgggcaacga ctggaataaa gcctataaaa aatctgcccg tgtcgttggt 60
gacgtaatcg gtaaatacca ccctcatggt gataccgctg tttacgacac cattgttcgt 120
atggcgcagc cattctcctt gcgttacatg ctggtagatg gtcagggtaa ctttggttct 180

<210> 92
<211> 26
<212> DNA
<213> Künstliche Sequenz

<220>
<223> Beschreibung der künstlichen Sequenz:Primer zur
      Klassifizierung von Enterobakterien

<400> 92
gaatccggga tacagtagag ggatag 26

<210> 93
<211> 26
<212> DNA
<213> Künstliche Sequenz

<220>
<223> Beschreibung der künstlichen Sequenz:Primer zur
      Klassifizierung von Enterobakterien

<400> 93
ccttaaacca accgtactgc aggcct 26

<210> 94
<211> 25
<212> DNA
<213> Künstliche Sequenz

<220>
<223> Beschreibung der künstlichen Sequenz:Primer zur
      Klassifizierung von Enterobakterien

<400> 94
gtatgcgatg tctgaactgg gcctg                                          25


<210> 95
<211> 25
<212> DNA
<213> Künstliche Sequenz

<220>
<223> Beschreibung der künstlichen Sequenz:Primer zur
      Klassifizierung von Enterobakterien

<400> 95
accgggattc ggtgtaacgc attgc                                          25


<210> 96
<211> 20
<212> DNA
<213> Künstliche Sequenz

<220>
<223> Beschreibung der künstlichen Sequenz:Primer zur
      Klassifizierung von Enterobakterien

<400> 96
gagctgttcc ttgtggaagg                                                20


<210> 97
<211> 20
<212> DNA
<213> Künstliche Sequenz

<220>
<223> Beschreibung der künstlichen Sequenz:Primer zur
      Klassifizierung von Enterobakterien

<400> 97
gagctgtttc ttgtggaggg                                                20


<210> 98
<211> 18
<212> DNA
<213> Künstliche Sequenz

<220>
<223> Beschreibung der künstlichen Sequenz:Primer zur
      Klassifizierung von Enterobakterien

<400> 98
ggtgttaagg atcttacc                                                  18


<210> 99
<211> 18

<212> DNA
<213> Künstliche Sequenz

<220>
<223> Beschreibung der künstlichen Sequenz:Primer zur
      Klassifizierung von Enterobakterien

<400> 99
ggtattaagg accttacc                                                    18


<210> 100
<211> 17
<212> DNA
<213> Künstliche Sequenz

<220>
<223> Beschreibung der künstlichen Sequenz:Primer zur
      Klassifizierung von Enterobakterien

<400> 100
ctgccgggca aactggc                                                     17


<210> 101
<211> 17
<212> DNA
<213> Künstliche Sequenz

<220>
<223> Beschreibung der künstlichen Sequenz:Primer zur
      Klassifizierung von Enterobakterien

<400> 101
ctgccgggca aactagc                                                     17


<210> 102
<211> 17
<212> DNA
<213> Künstliche Sequenz

<220>
<223> Beschreibung der künstlichen Sequenz:Primer zur
      Klassifizierung von Enterobakterien

<400> 102
acgttgagga ttttacc                                                     17


<210> 103
<211> 17
<212> DNA
<213> Künstliche Sequenz

<220>
<223> Beschreibung der künstlichen Sequenz:Primer zur
      Klassifizierung von Enterobakterien

<400> 103
acgttaagaa ttttacc                                                     17

## Patentansprüche

1. Verfahren zur genotypischen Klassifizierung von Bakterien, dadurch gekennzeichnet, daß
Sequenzen von Teilbereichen mindestens eines Gens ausgewählt aus der Gruppe gyrA, gyrB, parC und par E bestimmt werden und
die Klassifizierung durch Vergleich mit bekannten Sequenzen der jeweiligen Gene von Bakterien erfolgt, wobei
- im Falle des gyrA-Gens die Codons für die Aminosäuren Gly-81, Ser-83, Ala-84, Asp-87 gemäß der Numerierung des E.coli-gyrA-Gens für den Vergleich unberücksichtigt bleiben;
- im Falle des parC-Gens die Codons für die Aminosäuren Gly-78, Ser-80, Ala-81, Glu-84 gemäß der Numerierung des E.coli-parC-Gens für den Vergleich unberücksichtigt bleiben;
- im Falle des gyrB-Gens die Codons für die Aminosäuren Asp-426 und Lys-447 gemäß der Numerierung des E.coli-gyrB-Gens für den Vergleich unberücksichtigt bleiben und
- im Falle des parE-Gens die Codons für die Aminosäuren Asp-420 und Lys-441 gemäß der Numerierung des E.coli-parE-Gens für den Vergleich unberücksichtigt bleiben.

2. Verfahren nach Anspruch 1, dadurch gekennzeichnet, daß die Bestimmung der Sequenzen einen Amplifizierungsschritt für Teilbereiche der Gene umfaßt.

3. Verfahren nach Anspruch 1 oder 2, dadurch gekennzeichnet, daß die Bestimmung der Sequenzen eine Sequenzierungsreaktion umfaßt.

4. Verfahren nach einem der Ansprüche 1 bis 2, dadurch gekennzeichnet, daß das Verfahren die Hybridisierung der Sequenzen mit speziesspezifischen Oligonukleotidsonden und spektroskopischer Analyse der Hybridisierung umfaßt.

5. Nukleinsäure mit der Seq.ID-Nr. 1 bis 13 und Fragmente mit einer Länge von mindestes 8, bevorzugt 12 Nukleotiden.

6. Verwendung der Nukleinsäure mit der Seq.ID-Nr. 1 bis 13 und Fragmente mit einer Länge von mindestens 8 Nukleotiden als Sonden zur Klassifizierung von Bakterien.

7. Analysevorrichtung zur Klassifizierung von Bakterien, dadurch gekennzeichnet, daß die Analysevorrichtung Nukleinsäuren enthält, die spezifisch für Sequenzen von Teilbereichen von Genen, ausgewählt aus der Gruppe gyrA, gyrB, parC und parE von Bakterien-Spezies sind.

8. Zusammensetzung enthaltend mindestens zwei Nukleinsäuren, die spezifisch für Sequenzen von Teilbereichen von Genen, ausgewählt aus der Gruppe gyrA, gyrB, parC und parE von Bakterien-Spezies sind.

9. Verwendung der Analysevorrichtung gemäß Anspruch 7 oder der Zusammensetzung gemäß Anspruch 8 zur analytischen oder diagnostischen Klassifizierung von Bakterien.
